(19) Europäisches Patentamt European Patent Office Office européen des brevets

(11) **EP 2 716 282 A1**

(12) **EUROPEAN PATENT APPLICATION**

(43) Date of publication:
09.04.2014 Bulletin 2014/15

(51) Int Cl.:
*A61K 9/20* (2006.01)     *A61K 36/9066* (2006.01)

(21) Application number: 14150604.8

(22) Date of filing: 09.01.2014

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **College of Pharmacy of Taif University 21944 Taif (SA)**

(72) Inventors:
• **Al-Remawi, Mayyas**
  **21944 Taif (SA)**
• **Maghrabi, Ibrahim**
  **21944 Taif (SA)**

(74) Representative: **Scholz, Volker**
  **Boehmert & Boehmert**
  **Pettenkoferstrasse 20-22**
  **80336 München (DE)**

(54) **Controlled release pharmaceutical composition**

(57)     The present invention relates to a controlled release pharmaceutical composition comprising at least one pharmaceutically active agent and an excipient matrix, wherein the pharmaceutically active agent is substantially embedded in the excipient matrix and the excipient matrix is a mixture of a wax-like substance and chitosan; as well as to a method for its preparation.

Figure 1

**Description**

The present invention relates to a controlled release pharmaceutical composition and to its preparation method.

Oral controlled release delivery systems are numerous and nowadays become common and popular. These systems are more acceptable by the patients due to their prolonged pharmacological action. One interesting approach used to control the drug release is the gastro retentive systems (GRS). GRS can be produced by several ways, among the most interesting is by floating of the matrix tablets on the surface of gastric fluid. Floating systems can be achieved by mixing polymers with effervescent excipients, gel forming swellable hydrocolloids, hollow microspheres, super porous hydrogel systems, solid lipid systems and by the use of low density tablets, see, e.g. Shakti Dwivedi and Vikash Kumar. Floating Drug Delivery Systems- A Concept of Gastroretention Dosages Form. International Journal of Research in Pharmaceutical and Biomedical Sciences. Vol. 2(4) Oct - Dec 2011; Ashokkumar D and Guru Prasad Mohanta, Formulation and In vitro Evaluation of Ranitidine HCl Floating Tablets by Lipid Solid Dispersion Spray Drying Technique. International Journal of Research in Pharmaceutical and Biomedical Sciences. Vol. 3 (4) Oct - Dec 2012; A Streubel, J Siepmann, R Bodmeier. Floating microparticles based on low density foam powder Original Research Article International Journal of Pharmaceutics, Volume 241, Issue 2, 25 July 2002, Pages 279-292.

Low density tablets are an interesting class since they usually have lower density than aqueous medium which enables them to float on the surface of an aqueous medium. Many materials can be used to produce low density tablets such as gelucire, oils, glyceryl trimyristate, glyceryl palmitostearate, waxes, etc.

Wax unlike ordinary lipids is not readily absorbed through the gastrointestinal tract due to the difficulty in emulsification and highly non-polar nature.

Among the safest waxes and widely accepted as food stuff is bees wax (BW).

The techniques used in the production of solid lipid matrix systems are somewhat difficult and need sophisticated machines and possess low production rate due to the inability to use high speed compression machines. Therefore, there is an industrial need to convert the wax-like materials into compressible excipients.

Chitosan (CS) is a highly porous low density polymer. CS is a (poly(N-acetylglycosamine) which is partially deacetylated chitin. CS is the most abundant polysaccharide in nature second only to cellulose. It has a sugar backbone consisting of β-1,4 linked glucosamine with a high degree of N-acetylation, a structure very similar to that of cellulose; the only difference being the replacement of the hydroxyl by amino groups.

It is an object of the present invention to provide a controlled release pharmaceutical composition of low density being capable of providing a floating system in the stomach, while the pharmaceutical composition shall also allow to increase its porosity in the gastrointestinal tract to then also make the composition less dense with time.

It is also an object of the invention to provide a method for preparing such a pharmaceutical composition.

This object is achieved by a controlled release pharmaceutical composition comprising at least one pharmaceutically active agent and an excipient matrix, wherein the pharmaceutically active agent is substantially embedded in the excipient matrix and the excipient matrix is a mixture of a wax-like substance and chitosan.

It is preferred that the wax-like substance is a substance permitted for use in food, preferably bees wax.

Preferably, the wax-like substance is present in the excipient matrix in an amount of 1-50 wt.-%, based on the excipient matrix, preferably 2,5-40 wt.-%, preferably, 5-30 wt.-%, more preferably 10-30 wt.-%, most preferably, 10-20 wt.-%.

More preferably, the composition comprises other adjuvants selected from the group consisting of surfactants, alkalinizing agents, glidants, binders, lubricants, and mixtures thereof.

In a preferred embodiment, the composition is in the form of a tablet, capsule or granule.

Even preferred, the pharmaceutically active agent is curcumin.

A further object is achieved by a method for preparing a controlled release pharmaceutical composition according to any of the preceding claims, comprising the steps of: co-processing the wax-like substance and chitosan to provide an excipient matrix, and embedding the at least one pharmaceutical agent into the excipient matrix.

Finally, co-processing is achieved by adding chitosan powder into the melted wax-like substance, with stirring, optionally followed by sieving.

Surprisingly, it was found that the inventive controlled release pharmaceutical composition allows the provision of a hydrophobic water-insoluble wax-like phase in intimate contact with chitosan, to provide a co-processed excipient. This allows the provision of a low density excipient matrix capable of making floating systems in the stomach. Additionally, the chitosan dissolution which takes slowly place in the acidic medium of the stomach increases the porosity of the excipient matrix and further makes the excipient matrix less dense with time in the stomach. Such an excipient matrix can be used to develop floating pharmaceutical compositions of many drugs.

Further, by inclusion of the wax-like substance into the highly porous low density chitosan polymer, it is possible to convert such wax-like substances into compressible excipients.

Chitosan has, besides its low density, also the ability to absorb hydrophobic materials (lipids or wax-like substances). Further, chitosan is soluble in the stomach which may decrease the tablet weight with time, i.e. floating becomes easier. In addition, chitosan has a pH stabilizing ability in the microenvironment of the pharmaceutical composition.

In the present application, the term "wax-like substance" is to be understood as a chemical compound that is plastic near ambient temperatures. Such substances are also a type of lipid. Characteristically, they melt above 45°C to give a low viscosity liquid. Waxes and wax-like substances are insoluble in water, but soluble in organic, non-polar solvents. All waxes and wax-like substances are organic compounds, both synthetic and naturally occurring.

In another preferred embodiment, the excipient matrix contains a hydrophobic core substantially consisting of the wax-like substance and a hydrophilic shell substantially consisting of chitosan, suitable to float as a low density material to provide an excipient matrix suitable to release an embedded pharmaceutically active agent or drug slowly while the floated pharmaceutical composition slowly dissolves in the acidic medium of the stomach.

Further advantages and features of the present application can be taken from the following examples in conjunction with the attached drawing. In this drawing:

Figure 1: (A) FTIR of I) CS and BW (right) II) Different percentage of BW after embedding into CS (left). (B) ratio obtained after dividing the intensity obtained by FTIR for wave number 1668 cm-1 (CS) to 1749 cm-1 (BW)

Figure 2: DSC thermograms of different samples of BW after embedding into CS. Where, 1: BW, 2: CS, 3: CS to BW 9.5 to 0.5, 4: CS to BW 9 to 1, 5: CS to BW 8 to 2, 6: CS to BW 7 to 3, 7: CS to BW 6 to 4

Figure 3: Carr's index and Hausner's ratio determined for different CS to BW granules

Figure 4: Load displacement plots of 1 g sample mixtures of BW embedded in CS The ratio of Elastic work (EW) to Net compression work (NCW) obtained for different sample mixtures of BW embedded in CS

Figure 5: Viscosity versus shear rate (top) and viscosity versus temperature (bottom) plots for CS samples of 1% w/v containing different ratios of CS to BW dispersed in 0.1 M HCl

Figure 6: In-vitro dissolution profile of curcumin tablet (10 mg) in 500 ml at 50 rpm using USP apparatus 2 with dissolution medium containing 0.1 M HCl/0.1 % Tween 20 (A) and phosphate buffer pH 7.4/0.1 % Tween 20 (B) . Tablet contains silica, CS, curcumin, beeswax.

Figure 7: Composition of curcumin matrix tablet and the sequence of dissolution of the low density floating tablet inside the gastrointestinal tract with time

Curcumin [1,7-bis(4-hydroxy-3 methoxyphenyl)-1,6-heptadiene-3,5-dione], is a naturally yellow pigment extracted from the roots of *Curcuma longa,* a member of ginger family. Curcumin was selected as a model drug in the gastro-retentive system. There are many reasons to develop curcumin as a gastro retentive system (GRS) including: Curcumin is poorly absorbed from the gastrointestinal tract (GIT) and thus increasing the gastrointestinal resident time could increase the time of absorption.

Also, curcumin is stable in slightly acidic conditions of the stomach and upper part of small intestines, while at alkaline pH curcumin will degrade.

Moreover, local or sustained delivery of curcumin to the stomach and upper small intestine is useful in the treatment of stomach diseases like peptic ulcers caused by H. Pylori or stomach cancers because curcumin is also considered a powerful stomach anticancer agent.

In addition to the previous reasons, curcumin is increasingly attracting much attention due to its wide range pharmacological actions such as anti-inflammatory, antioxidant, anti-infectious, anti-parasitic and antitumor activities.

## Examples

## Example 1

## Production of granules of BW embedded in CS

CS powder was heated at a temperature of 75°C for 15 min with stirring. BW was melted at a temperature of 75°C for 15 min. CS was added geometrically to melted BW while mixing and keeping the temperature at 75 °C for 10 min. The particles were then sieved while hot. Then they were cooled down to room temperature and then re-sieved again. The prepared CS/BW mixtures are summarized in Table 1.

Table 1: Composition of granules produced by CS and BW

| formula | Chitosan (CS) (g) | Bees wax (BW) (g) | Ratio CS:BW | Percentage BW (%) |
|---|---|---|---|---|
| F1 | 100 | 0 | 10:0 | 0 |
| F2 | 97.5 | 2.5 | 9.75:0.25 | 2.5 |
| F3 | 95 | 5 | 9.5:0.5 | 5 |
| F4 | 90 | 10 | 9:1 | 10 |
| F5 | 80 | 20 | 8:2 | 20 |
| F6 | 70 | 30 | 7:3 | 30 |
| F7 | 60 | 40 | 6:4 | 40 |
| F8 | 50 | 50 | 5:5 | 50 |

**Example 2**

**Characteristics of BW/ CS (granules) by FTIR**

Around 5 mg sample of each CS/BW mixture was mixed with 100 mg KBr, compressed using a hand press to produce thin tablet and scanned using FTIR (IR Prestige 21, Shimadzu, Japan).

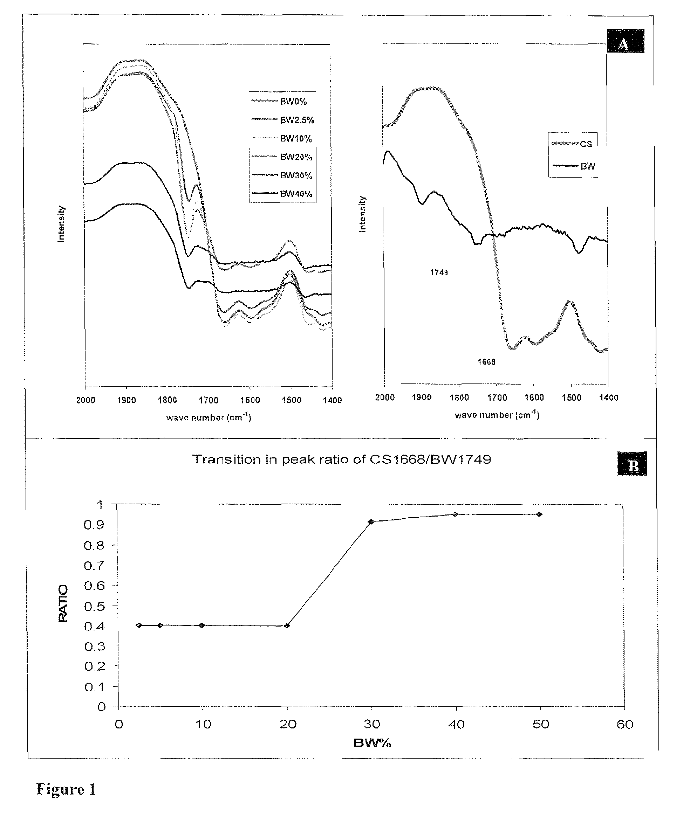

The FTIR of all mixtures were scanned as depicted in left hand side of Figure 1.

There is a peak at 1668 cm-1 indicating the presence of CS and another peak for BW at 1749 cm-1 as shown in the right hand side of Figure 1, A.

The peak ratio of CS at1668 cm-1 to BW at 1749 cm-1 was calculated and plotted against percentage BW in the tablet. Results indicated that 0-20% BW has similar ratio while above 20% a transition occurs and another constant ratio appeared as shown in Figure 1, B. Thus as an expectation, the excipient of BW and CS shall have two different characteristics depending on the ratio of BW to CS. The transition occurred above 20% BW or above BW:CS 8:2.

This could be explained as follows: CS as a highly porous polymer initially absorbs BW inside its intimate cavities until its fully saturated where BW dominates and starts covering CS surface.

**Example 3**

**Characteristics of BW/ CS (granules) by Differential Scanning Calorimeter (DSC)**

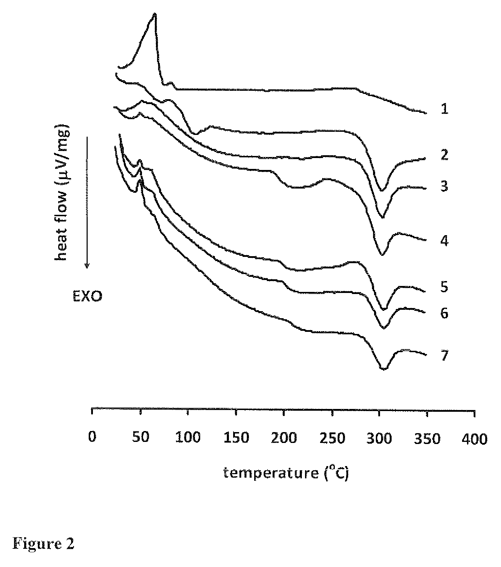

A sample of 5 mg CS/BW granules were put in a DSC pan and scanned for its thermal behavior. The starting temperature was 25 ending with 300 °C at a heating rate of 10 °C min$^{-1}$ in an open aluminum pan with an empty pan as a reference using differential scanning calorimeter, DSC (STA 449 F1 Jupiter® NETZSCH, Germany). There is a heat flux transition occurred above 20% BW or above BW:CS 8:2, as shown in Figure 2.

**Example 4: Flowability of BW/CS granules**

Flowability was measured using simple methods of Carrs index (C) and Hausner ratio (H) as in equations 1 and 2

$$C = 100 \times \left(1 - \frac{\rho_B}{\rho_T}\right) \qquad\qquad 1$$

$$H = \frac{\rho_T}{\rho_B} \qquad\qquad 2$$

Where $\rho_B$ and $\rho_T$ are the tap and bulk densities calculated based on weight of material per envelope volume before and after tapping, respectively.

A Carr's index greater than 25 is considered to be an indication of poor flowability, and below 15, of good flowability. While, a Hausner ratio greater than 1.25 is considered to be an indication of poor flowability, see also Kanig, Joseph L.; Lachman, Leon; Lieberman, Herbert A. (1986). The Theory and Practice of Industrial Pharmacy (3 ed.).

Philadelphia: Lea & Febiger.

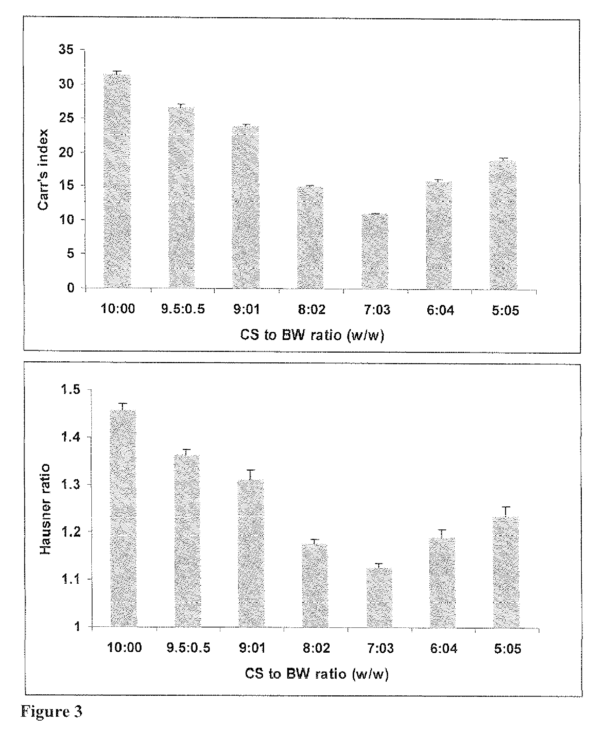

Figure 3 summaries the results of Carr's index and Hausner ratios of CS/BW mixtures.

The best flow was obtained in the range of BW 20-30% in CS/BW mixture.

**Example 5:**

**Compactability of BW/ CS granules**

1 gram sample of each CS/BW mixture was compacted using Instron at 20 Kgf using 15 mm circular die. Load displacement plots of each CS/BW mixture were obtained. Then, the net work of compaction and elastic work were calculated for each plot.

NCW (Net compaction work, J/g) is the work of the material to be compacted and EW (Elastic work, J/g) describes the energy associated with the expansion during unloading due to the elasticity of the compact were determined using pressure displacement plots.

The ratio between NCW and EW was calculated according to C. Pontiera, E. Championa, M. Vianab, D. Chuliab, D. Bernache-Assollanta. Use of cycles of compression to characterize the behaviour of apatitic phosphate powders. Journal of the European Ceramic Society 22 (2002) 1205-1216.

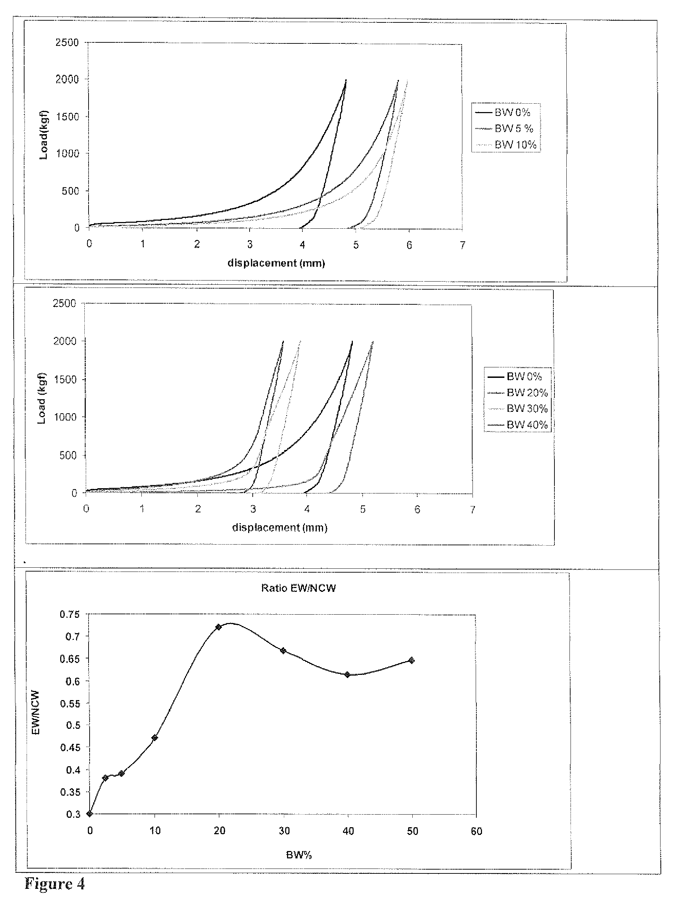

Figure 4 shows the load displacement plots of 1 g sample mixtures of BW embedded in CS. The ratio of Elastic work (EW) to Net compression work (NCW) obtained for different sample mixtures of BW embedded in CS. Obviously, the maximum ratio was obtained at BW 20% or in other words at this ratio a transition occurred.

**Example 5: Theological properties in acidic conditions**

1 gram CS/BW mixture was dispersed in 100 ml 0.1 M HCl and homogenized at 2000 rpm for 2 min, then the samples were sonicated for 1 min. 1.5 ml sample was placed on the plate of rheometer (Bohlin CVO Rheometer, Malvern, UK) and viscosity was measured at shear rate (240-0.1 $s^{-1}$) at a constant temperature of 25°C. Also, the viscosity was measured at a constant shear rate (100 $s^{-1}$) and temperature of 25 to 55°C.

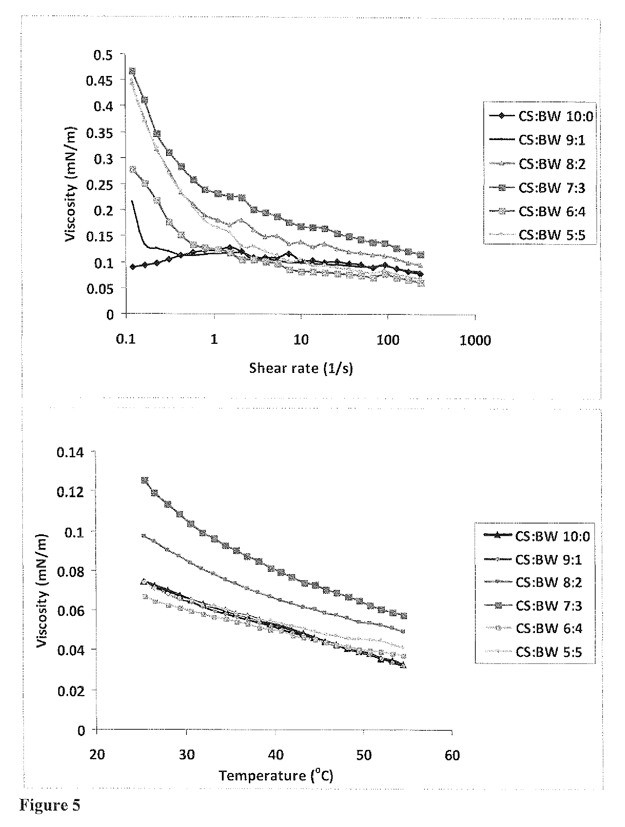

Figure 5 shows that at BW 20 to 30 % resulted in higher viscosity compared with other ratios at all shear rates. Also, similar results were found at all temperatures used.

**Example 6:**

**Formulation of curcumin loaded silica particles tablets and testing their floating power**

An amount of 1 g of curcumin powder and 3 g silica particles (containing 0.25% w/w Tween 80) were added gradually and triturated using pestle and mortar. Then, 3 g of different CS/BW granules were added and mixed gently and compressed using Universal Testing Machine (Instron, USA) at a constant force 6 mm circular die. The tablet weight was 70 mg containing 10 mg curcumin per each tablet. The tablet hardness was measured (hardness tester, Erweka, Germany).

The produced curcumin tablets of each CS/BW mixture were put in 500 ml 0.1 M HCl or USP phosphate buffer 7.4 at 37 °C in USP dissolution apparatus (paddle) at 50 rpm . The time required for the tablets to float on the surface was determined.

Table 2 shows the tablet hardness and floating time of different curcumin tablets produced. The addition of BW was found to produce floating tablet up to a percentage of 20%. Tablets produced through compaction via Instron resulted in an acceptable compact with suitable hardness (40-70 N) for such small sized tablets. However, tablets containing BW above 20 % w/w did not float upon observing them for a period of 10 min.

Table 2: Tablet hardness and floating time of curcumin tablets

| Curcumin Formula | Excipient CS:BW ratio | Tablet hardness (N) | Floating time in 0.1 M HCl (s) | Floating time in Ph Buffer 7.4 (s) |
|---|---|---|---|---|
| CF1 | 10:0 | 75 | NF | NF |
| CF2 | 9.75:0.25 | 40 | **20** | **17** |
| CF3 | 9:1 | 42 | **2** | **4** |
| CF4 | **8:2** | **44** | **2** | **2** |
| CF5 | 7:3 | 62 | NF | NF |
| CF6 | 6:4 | 57 | NF | NF |

(continued)

| Curcumin Formula | Excipient CS:BW ratio | Tablet hardness (N) | Floating time in 0.1 M HCl (s) | Floating time in Ph Buffer 7.4 (s) |
|---|---|---|---|---|
| CF7 | 5:5 | 59s | NF | NF |

NF: Non floating tablets Floating behavior was similar in both dissolution media i.e. 0.1 M HCl and phosphate buffer pH 7.4

**Example 7: in vitro dissolution of curcumin preparation in 0.1 M HCl and in phosphate buffer pH 7.4**

The prepared tablets in Table 2 were also subjected to USP dissolution test with the same dissolution conditions of Example 6 (except each 0.1% Tween 20 was added per every dissolution medium). 5 ml sample was withdrawn at different time interval and replaced with same volume. The sample was analyzed using UV-Visible spectrophotometer (UV1800, Shimadzu, Japan). The analysis was determined after establishing a linear calibration curve of curcumin (0.1 M HCl, $R^2$ =0.9996, conc. range 0.3-3 mg/dL at 424 nm) and (phosphate buffer pH 7.4 , $R^2$= 0.9999, conc. range 0.02-1.5 mg/dL).

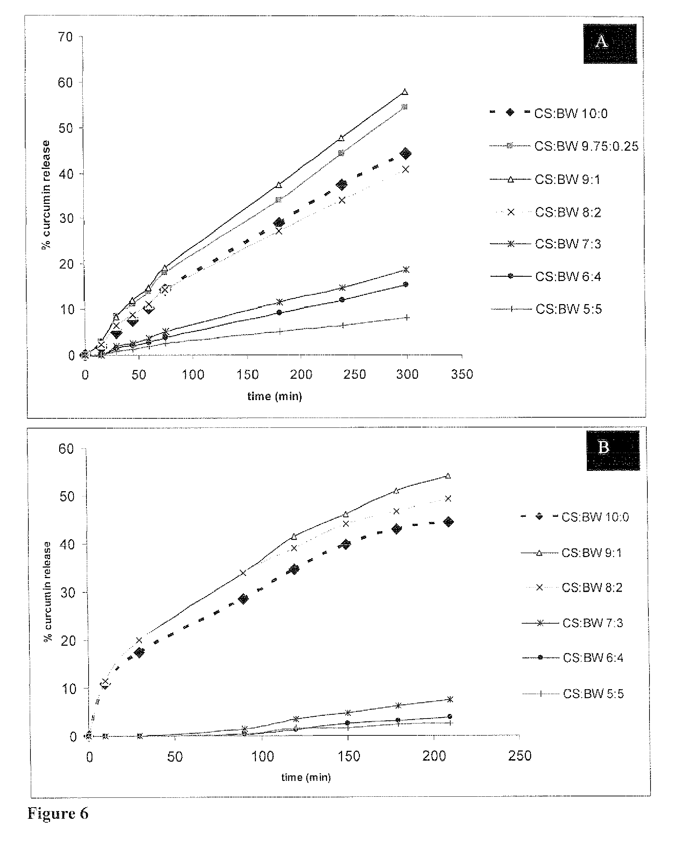

Figure 6 shows the release of curcumin out of the floating BW/CS tablet in acidic and alkaline conditions. The release of floating BW/CS tablets was relatively higher than that of the non-floating tablets. The transition in release pattern also occurred at BW 20%.

Thus, it could be concluded that for the development of floating BW/CS mixture the proper ratio should be preferably less than 30% for BW. More specifically, in the range 10-30% w/w.

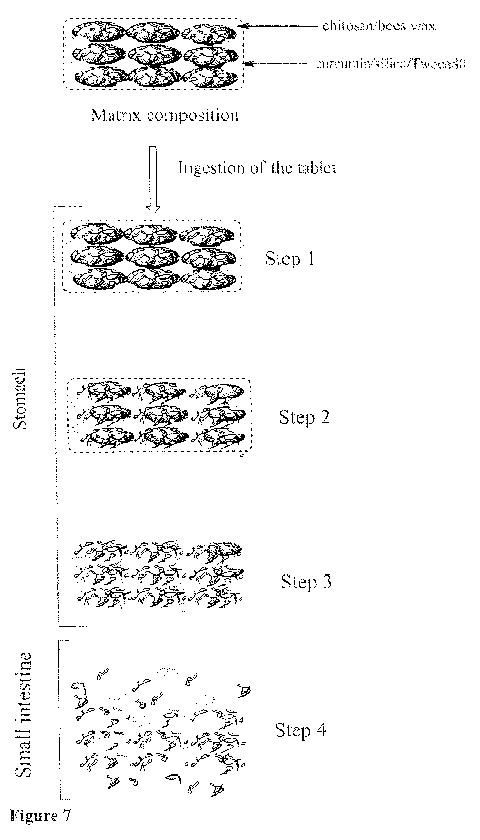

Based on the release study observations, the behavior of the floating tablet can be described as in Figure 7.

Step 1: the Tablet will float inside the stomach due to low density excipient (CS/BW) and drug start releasing.

Step 2: The tablet preserve floating due to losing CS with time (dissolution) and tablet density is decreasing with time.

Step 3: Highly porous tablet will be formed when CS is completely dissolved, tablet will start fragmentation and curcumin release will be exaggerated (disintegration).

Step 4: Fragments of tablet enter the small intestine and the remaining curcumin will be released in the small intestine.

The features disclosed in the foregoing description, in the claims and in the accompanying drawings may both separately and in any combination thereof be material for realizing the invention in diverse forms thereof.

**Claims**

1. Controlled release pharmaceutical composition comprising at least one pharmaceutically active agent and an excipient matrix, wherein the pharmaceutically active agent is substantially embedded in the excipient matrix and the excipient matrix is a mixture of a wax-like substance and chitosan.

2. Pharmaceutical composition according to claim 1, wherein the wax-like substance is a substance permitted for use in food, preferably bees wax.

3. Pharmaceutical composition according to claim 1 or 2, wherein the wax-like substance is present in the excipient matrix in an amount of 1-50 wt.-%, based on the excipient matrix, preferably 2,5-40 wt.-%, preferably, 5-30 wt.-%, more preferably 10-30 wt.-%, most preferably, 10-20 wt.-%.

4. Pharmaceutical composition according to any of the preceding claims, which composition comprises other adjuvants selected from the group consisting of surfactants, alkalinizing agents, glidants, binders, lubricants, and mixtures thereof.

5. Pharmaceutical composition according to any of the preceding claims, wherein the composition is in the form of a tablet, capsule or granule.

6. Pharmaceutical composition according to any of the preceding claims, wherein the pharmaceutically active agent is curcumin.

7. Method for preparing a controlled release pharmaceutical composition according to any of the preceding claims, comprising the steps of:

co-processing the wax-like substance and chitosan to provide an excipient matrix, and
embedding the at least one pharmaceutical agent into the excipient matrix.

8. Method according to claim 7, wherein the co-processing is achieved by adding chitosan powder into the melted wax-like substance, with stirring, optionally followed by sieving.

Figure 1

Figure 2

Figure 3

Figure 4

Figure 5

Figure 6

Figure 7

Europäisches
Patentamt

European
Patent Office

Office européen
des brevets

**EUROPEAN SEARCH REPORT**

Application Number

EP 14 15 0604

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 391 294 B1 (DETTMAR PETER WILLIAM [GB] ET AL) 21 May 2002 (2002-05-21) * examples 23-25 * * column 5, lines 48-67 * * column 6, lines 33-59 * ----- | 1,2,4,5, 7,8 | INV. A61K9/20 A61K36/9066 |
| X | US 2008/175957 A1 (HORGAN MONIKA BARBARA [US] ET AL) 24 July 2008 (2008-07-24) * page 1, paragraph 0002 * * page 3, paragraphs 0047, 0054, 0055 * * page 4, paragraph 0058-0066 * * examples 2, 4, 9, 11 * * claims 1, 2, 9, 19, 21, 25, 30-32 * ----- | 1-6 | |
| X | EP 2 042 166 A1 (JORDANIAN PHARMACEUTICAL MFG [JO]) 1 April 2009 (2009-04-01) * example 3 * * claims * ----- | 1,2,4,5 | |

TECHNICAL FIELDS
SEARCHED    (IPC)

A61K

The present search report has been drawn up for all claims

| Place of search | Date of completion of the search | Examiner |
|---|---|---|
| The Hague | 4 March 2014 | van de Wetering, P |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&: member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 14 15 0604

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2014

| Patent document cited in search report | | Publication date | Patent family member(s) | | | Publication date |
|---|---|---|---|---|---|---|
| US 6391294 | B1 | 21-05-2002 | AR | 016850 | A1 | 01-08-2001 |
| | | | AT | 244562 | T | 15-07-2003 |
| | | | AU | 737714 | B2 | 30-08-2001 |
| | | | AU | 8738998 | A | 16-03-1999 |
| | | | BR | 9811245 | A | 18-07-2000 |
| | | | CA | 2301165 | A1 | 04-03-1999 |
| | | | CN | 1267216 | A | 20-09-2000 |
| | | | DE | 69816307 | D1 | 14-08-2003 |
| | | | DE | 69816307 | T2 | 27-05-2004 |
| | | | EP | 1007015 | A1 | 14-06-2000 |
| | | | ES | 2198062 | T3 | 16-01-2004 |
| | | | GB | 2328443 | A | 24-02-1999 |
| | | | HU | 0003602 | A2 | 28-03-2001 |
| | | | JP | 2001513549 | A | 04-09-2001 |
| | | | PL | 338701 | A1 | 20-11-2000 |
| | | | US | 6391294 | B1 | 21-05-2002 |
| | | | WO | 9909962 | A1 | 04-03-1999 |
| US 2008175957 | A1 | 24-07-2008 | AR | 064953 | A1 | 06-05-2009 |
| | | | AU | 2008206670 | A1 | 24-07-2008 |
| | | | BR | PI0806803 | A2 | 13-09-2011 |
| | | | CA | 2674422 | A1 | 24-07-2008 |
| | | | EP | 2124608 | A2 | 02-12-2009 |
| | | | JP | 2010516246 | A | 20-05-2010 |
| | | | US | 2008175957 | A1 | 24-07-2008 |
| | | | WO | 2008087607 | A2 | 24-07-2008 |
| EP 2042166 | A1 | 01-04-2009 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SHAKTI DWIVEDI ; VIKASH KUMAR.** Floating Drug Delivery Systems- A Concept of Gastroretention Dosages Form. *International Journal of Research in Pharmaceutical and Biomedical Sciences,* October 2011, vol. 2 (4
- **ASHOKKUMAR D ; GURU PRASAD MOHANTA.** Formulation and In vitro Evaluation of Ranitidine HCl Floating Tablets by Lipid Solid Dispersion Spray Drying Technique. *International Journal of Research in Pharmaceutical and Biomedical Sciences,* October 2012, vol. 3 (4
- **A STREUBEL ; J SIEPMANN ; R BODMEIER.** Floating microparticles based on low density foam powder Original Research Article. *International Journal of Pharmaceutics,* 25 July 2002, vol. 241 (2), 279-292
- **KANIG, JOSEPH L. ; LACHMAN, LEON ; LIEBERMAN, HERBERT A.** The Theory and Practice of Industrial Pharmacy. Lea & Febiger, 1986
- **C. PONTIERA ; E. CHAMPIONA ; M. VIANAB ; D. CHULIAB ; D. BERNACHE-ASSOLLANTA.** Use of cycles of compression to characterize the behaviour of apatitic phosphate powders. *Journal of the European Ceramic Society,* 2002, vol. 22, 1205-1216